# EUROPEAN PATENT SPECIFICATION

(11) **EP 0 362 288 B1**
(45) Date of publication and mention of the grant of the patent: **11.01.1995**
(21) Application number: 88905516.6
(22) Date of filing: 27.05.1988
(51) Int. Cl.: C12N 15/38, C12P 21/02, C12N 1/21, A61K 39/245, G01N 33/569, C12Q 1/70

(54) **EXPRESSION OF IMMUNOLOGICALLY ACTIVE PROTEINS OF HUMAN B-LYMPHOTROPIC VIRUS**
EXPRESSION DER IMMUNOAKTIVEN PROTEINE VON MENSCHLICHEM B-LYMPHOTROPIKVIRUS
EXPRESSION DE PROTEINES IMMUNOLOGIQUEMENT ACTIVES DU VIRUS B-LYMPHOTROPE HUMAIN

(30) Priority: 01.06.1987 US 56963
(43) Date of publication of application: 11.04.1990
(73) Proprietor: BAYLOR COLLEGE OF MEDICINE, Houston, TX 77030 (US); THE GOVERNMENT OF THE UNITED STATES OF AMERICA as represented by THE SECRETARY of the DEPARTMENT OF HEALTH AND HUMAN SERVICES, Washington, DC 20201 (US)
(72) Inventor: CHANG, Nancy, T., Houston, TX 77025 (US); CHANG, Tse, Wen, Houston, TX 77025 (US); FUNG, Michael, Sek-Chung, Houston, TX 77025 (US); FUNG, Ming-Chiu, Houston, TX 77025 (US); GALLO, Robert, C., Bethesda, MD 20817 (US); WONG-STAAL, Flossie, Rockville, MD 20850 (US)
(74) Representative: Holdcroft, James Gerald, Dr.
(86) International application number: US8801807
(87) International publication number: WO8809814

(56) References cited:
- WO-A-88/00980
- WO-A-88/01305
- Science, vol. 234, 31 October 1986, S.Z. Salahuddin et al., pp. 596-601
- Science, vol. 234, 31 October 1986, S.F. Josephs et al., pp. 601-603
- Science, vol. 228, 5 April 1985, N.T. Chang et al., pp. 93-96
- Biological Abstracts, vol. 85, no. 5, 1988, (Philadelphia, Pennsylvania, US), P. Biberfeld et al., p. AB-616, abstract no. 48824

## Description

### Background

Human B-lymphotropic virus (HBLV)is a DNA virus isolated recently from several patients with certain lymphoproliferative disease. S.Z. Salahuddin, et al., Science 234, 596 (1986); S.F. Josephs et al., Science 234, 601 (1986). Morphological studies using electron microscopy have classified HBLV in the herpesvirus family. Biological studies of the virus in cultured cells, however, revealed that HBLV has many characteristics which distinguish it from other known transforming human and primate herpes-viruses. HBLV has a strict trophism and infects only the freshly isolated B cells. It has cytopathic properties and kills the target lymphocytes within about two weeks after infection. Furthermore, immunochemical studies show that antibodies or DNA probes specific for the various known herpes-viruses such as Epstein Barr virus (EBV) and human cytomegalovirus (CHMV) have little crossreactivity with HBLV.

The HBLV genome consists of a DNA duplex of greater than 60 X 10⁶ daltons in size. Replication of the DNA occurs in the nucleus of the infected cells. The virion DNA is contained within an icosahedral capsid composed of 162 capsomeres. The study of the structure and organization of HBLV has been difficult due to the lack of a chronically infected HBLV producing cell line. Small amounts of HBLV virion particles have been purified from infected core blood cell culture supernatants. DNA isolated from the purified virus was digested with HindIII and cloned into bacterial plasmids. One such clone designated as pZVH14 was partially characterized. See Josephs et al., supra.

With the discovery of HBLV and the possible involvement of this virus in disease, a need arises for tests which can detect the presence of the virus. Undoubtedly, the HBLV is transmissible through blood products. In order to prevent transmission of the virus, it is important to be able to screen blood products to ascertain whether blood products have been obtained from a donor that has been infected with HBLV.

The presence of antibody against a virus in blood is an indicator of exposure to virus. Immunochemical tests for antibody against HBLV might be based upon the use of a whole virus or upon the use of isolated viral components as antigen for detection of the antibody. However, because a compatible host cell line for propagation of the virus has not been developed, the production of large quantities of virus to supply reagent for use in tests may be difficult.

### Disclosure of the Invention

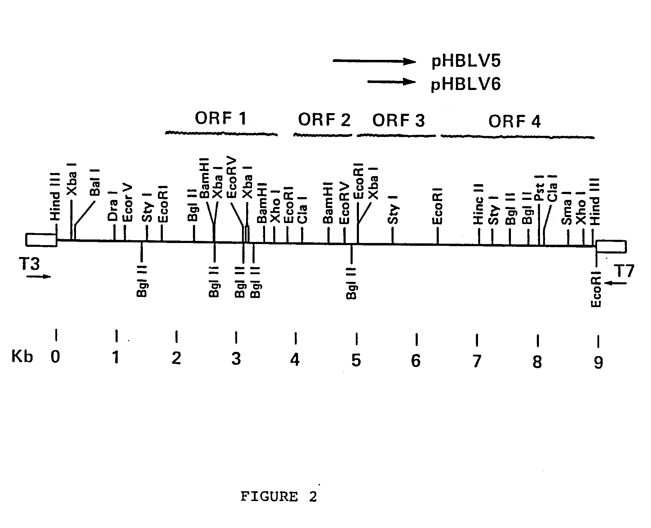

This invention pertains to an isolated HBLV protein which is immunoreactive with antibody against HBLV comprising an amino acid sequence encoded by (1) the ORF3 of Fig. 2, or (2) immunological equivalents thereof which are created by deletion, insertion or substitution of the ORF3 of figure 2. The invention is based upon the discovery of certain open reading frame regions of the HBLV genome which encode proteins that react with anti-HBLV antibodies in the serum HBLV infected individuals. The immunoreactive recombinant HBLV proteins can be expressed by cloned DNA segments of the HBLV genome in prokaryotic or eukaryotic expression systems to provide quantities of protein for use in immunochemical assays for HBLV detection. The proteins can be used in assays of various formats to detect antibodies against HBLV proteins in biological fluids such as blood and blood components.

DNA sequences encoding immunoreactice HBLV proteins were identified by a shot gun cloning technique in which fragments of the HBLV genome were randomly cloned as fused genes into E. coli and expressed as a β-galactosidase fusion proteins. At least two clones generated in this manner were found to produce proteins which were strongly immunoreactive with patient sera. The HBLV DNA segments of the clones were sequenced and based upon this information, the HBLV-derived portion of the fusion proteins was determined to be encoded by an open reading frame segment of the HBLV genome (designated ORF3). Thus, the region of the HBLV genome was determined to encode an immunogenic HBLV protein.

The cloning/expression protocol used herein (described more fully below) can be used to identify additional open reading frame regions of the HBLV genome which might encode immunoreactive proteins.

Immunochemical assays employing the HBLV proteins of this invention for detection of antibody against HBLV can take several forms, including immunometric assays and antigen sandwich assays. The preferred type of assay is a solid phase immunometric (double antibody) assay. Purified recombinant HBLV protein is immobilized by attaching it to solid phase to form an antigen immunoadsorbent. The immunoadsorbent is used to adsorb anti-HBLV antibody in a sample of biological fluid. The adsorbed anti-HBLV antibody is detected with an anti-(human IgG) antibody which is labeled radioisotopically, enzymatically, fluorometrically or in other ways. This second antibody, directed generally against human IgG, binds to anti-HBLV antibody adsorbed to the immunoadsorbent and generates a detectable signal which can be evaluated as an indication of the presence of anti-HBLV antibody in the sample.

The recombinant HBLV proteins of this invention can be used as immunogens for administration to a host animal or individual. In general, the immunogen compositions comprise an immunoactive HBLV protein and a physiologically acceptable vehicle. The immunogen compositions can be used to immunize a host animal for production of monoclonal or polyclonal antibody against HBLV. In addition, immunogen compositions can be used to stimulate an immune response to HBLV in an individual.

### Brief Description of the Figures

Figure 1(a) shows the expression vector pMLB1111 and the site for HBLV DNA insertion.

Figure 1(b) shows the nucleotide and amino acid sequence for the polylinker region of pMLB1111.

Figure 2 shows the open reading frame (ORF) regions of HBLV subgenomic DNA.

Figure 3 shows the DNA sequence of the HBLV-derived insert of clone pHBLV6.

Figure 4 shows SDS polyacrylamide gel analysis and Western blot of HBLV-galactosidase fusion proteins.

### Detailed Description of the Invention

A combined cloning/expression protocol was employed to identify HBLV genes encoding proteins which are reactive with antibody against HBLV. The protocol was based upon the principle of "insertional activation". See e.g., Berman et al., U.S. Patent No. 4,503,142. An expression vector was employed which contained a LacZ gene that has a frameshift mutation (resulting from the insertion of a polylinker DNA segment) at the N-terminal region of a the gene so that it is inactivated. Insertion of open reading frame DNA segments into this region can restore the correct reading frame and result in activation of β-galactosidase. When this occurs, a fusion protein is expressed which is made up of the protein encoded by the inserted DNA linked to a functional β-galactosidase C-terminal region. Thus, clones which express HBLV DNA as a fusion protein have β-galactosidase activity and can be identified based upon this phenotype. The expressed proteins can then be examined for immunoreactivity with HBLV antibody by, for example, the Western blot technique.

This procedure was used to identify DNA sequence of HBLV genomic DNA which encode immunoreactive proteins. A cloned 9kB subgenomic fragment of HBLV genome was obtained from Dr. Robert Gallo of the National Cancer Institute (pZVH14). Two methods were used to generate random DNA subfragments from the 9kb HBLV DNA subgenomic fragment: (1) DNase Bal31 was used to digest the subgenomic DNA derived from into approximately 1000 base-pair regions, and (2)the HBLV subgenomic DNA was sheared to about 300 bp to 1000 bp fragments by sonication. These DNA fragments were inserted in the expression vector pMB1111 and HBLV DNA sequences were expressed in E. coli as proteins fused to β-galactosidase. Sera from patients infected with HBLV containing antibodies to HBLV were then used to screen for immunoreactive fusion proteins. Two expression plasmids (pHBLV5 and pHBLV6) were isolated that specified fusion proteins that reacted in the western blot analysis with the patient sera. The HBLV DNA sequences represented in the expression plasmids were mapped within a single open reading frame designated ORF3 on the HBLV genome.

The method described above and in further detail in the Exemplification below can be employed to identify other sequences of the HBLV genome which encode immunologically active proteins.

Immunoreactive HBLV protein for use in diagnostic and therapeutic techniques can be prepared by several different methods. For one, HBLV DNA sequences which encode immunoreactive proteins can be expressed in prokaryotic or eukaryotic expression systems to yield the protein. HBLV sequences for expression can be obtained from genomic HBLV DNA.

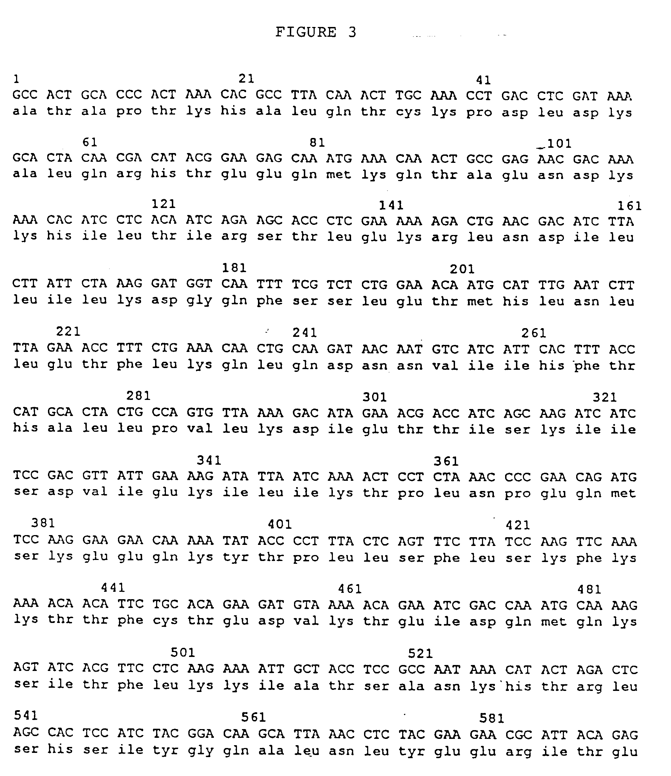

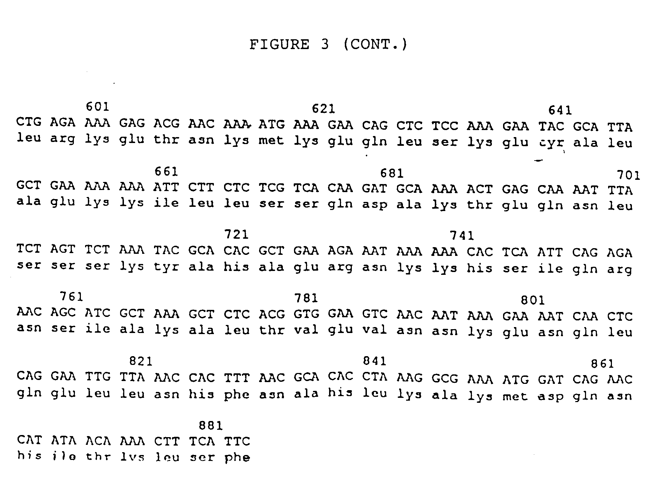

The immunoreactive HBLV proteins are encoded by open reading frame region (ORF3) of the 9kB HindIII subgenomic fragment of HBLV shown in figure 2. Especially preferred is the HBLV protein encoded by ORF3 whose amino acid sequences (and DNA sequence) is given in figure 3. (This protein is expressed as a fusion protein by pHBLV6). As noted, the protein encoded by this sequence is immunoreactive with antibody in serum of patients exposed to HBLV.

The DNA shown in figure 3 can be obtained from genomic HBLV DNA or it can be synthesized as described below.

The HBLV protein encoded by ORF3 is a part of the naturally occurring form of an HBLV antigen. Thus, the entire gene which encodes the antigen probably encompasses or overlaps the identified sequence in Figure 3. This gene can be cloned and expressed and the product may exhibit equivalent or even superior reactivity with HBLV patient sera. In addition, other segments of HBLV DNA which encompass the polypeptide coding region or those which overlap this region and DNA segments which are modified by deletion, insertion or substitution of nucleotides may also yield HBLV proteins or polypeptides which exhibit the immunological properties: all such "immunological equivalents" are embraced by this invention.

Although a cell line for propagation of the HBLV is not available, sufficient genomic HBLV DNA can be prepared as described by Josephs, S.F. et al., Science 234,601 (1986), the teachings of which are incorporated by reference herein. The genomic DNA can be subcloned as, for example, was done by Josephs et al. supra. The desired HBLV sequence can be excised from the genomic DNA (generally by restriction enzyme digestion), and cloned into other suitable cloning vehicles such as pUC or pEMBL. Probes can be prepared from HBLV DNA to identify clones containing desired insert in situ. For example, probes for the ORF3 region of the HBLV genome can be prepared based upon the DNA sequence given in Figure 3. If desired, sequences encoding immunoreactive proteins can be subcloned to remove unnecessary or unwanted sequences and to add restriction sites to the termini to facilitate cloning into an expression vector. The HBLV sequence can then be inserted into an expression vector, preferably one which can express the encoded protein at high levels in the particular host, to form a recombinant expression vector containing the HBLV sequence. The transformed host cell is cultured in an appropriate culture medium and the expressed HBLV protein is then isolated from the host cell.

Instead of isolating the HBLV DNA segments from genomic (or cloned subgenomic) DNA. HBLV DNA segments encoding antigenic regions can be synthesized chemically. Several techniques are available for synthesizing DNA of desired nucleotide sequences. See, e.g., Matteucci et al., J. Am. Chem. Soc. (1981) 103:3185; Alvarado-Urbina et al., Science (1980) 214:270. A preferred technique for synthesis of DNA segments is the β-cyanoethyl phosphoramidite chemistry. See e.g., Sinha, N.D. et al., Nucleic Acids Research 13, 4539 (1984). Figure 3 provides the sequence information of preferred proteins. Synthesized DNA can be adapted and inserted into an expression vector which can be used to transform vector compatible host cells and provide for expression of the encoded gene product as discussed above.

The expression systems for expression of immunoactive HBLV protein can be prokaryotic or eukaryotic. As mentioned, preferred systems are those by which the protein can be expressed at high levels. The protein can be expressed under control of an inducible promoter in eukaryotic cells. Some examples of promoter/cell systems are:
1) SV40 promoter/CHO cells;
2) metallothionine promoter/bovine, papilloma virus/murine C127 cells; and
3) adenovirus late promoter/COS-1 cells.
Additionally, recombinant vaccinia virus can be used to express the protein (as well as provide a live vaccine as described below). Other modes of expressing the HBLV proteins are known in the art.

When expressed as heterologous protein in a host cell system, any of several purification techniques can be used to purify the recombinant HBLV proteins. See e.g. Olson, U.S. Patent 4,518,526. In addition, affinity purification techniques as described below can be employed. For use in immunoassays, the HBLV Protein must be purified to substantial immunological purity, that is, the preparation should be substantially free of host cell contaminants which might be reactive with other antibody in human sera. Such contaminants could yield a high level of false positive results which would detract from the accuracy of the assay.

The HBLV proteins encoded by the identified antigenic regions of HBLV genome can be synthesized de novo chemically. For example, as pointed out the amino acid sequence of the protein expressed by the clone pHBLV6 is shown in Figure 2; the protein can be synthesized by the solid phase procedure of Merrifield. Based upon the sequence information provided, smaller immunologically active peptides can be designed. Chemical synthesis of such peptides would be simpler and less costly.

HBLV proteins or polypeptides (such as the protein encoded by ORF3) which are immunologically reactive with HBLV sera are immunogenic viral proteins and thus, polyclonal or monoclonal antibody can be prepared against these recombinant HBLV proteins. These antibodies can be used to purify the proteins (e.g. by immunoaffinity purification). In addition, the antibodies can be used in immunochemical assays for the direct detection of HBLV in biological fluids (as opposed to the detection of antibody against HBLV as described below). Monoclonal antibodies against the HBLV proteins can be produced by the standard somatic cell hybridization techniques of Kohler and Milstein. Nature 256:495 (1975). Polyclonal antibodies can be produced by conventional techniques. Immunogen compositions for stimulating the production of antibodies against HBLV comprising the immunoreactive HBLV proteins of this invention are described below.

Immunochemical assays employing the HBLV protein for detection of antibody against the virus in a biological fluid can take a variety of forms. The preferred type is a solid phase immunometric assay. In assays of this type, a purified recombinant HBLV protein is immobilized on a solid phase to form an antigen-immunoadsorbent. The immunoadsorbent is incubated with the sample to be tested. The duration and conditions of the incubation, those appropriate for the formation of the antigen-antibody complex. The immunoadsorbent is then separated from the sample and a labeled anti-(human IgG) antibody is used to detect human anti-HTLV-III antibody bound to the immunoadsorbent. The amount of label associated with the immunoadsorbent is compared to positive and negative controls to assess the presence of absence of anti-HTLV-III antibody.

The immunoadsorbent can be prepared by adsorbing or coupling purified HBLV protein to a solid phase. Various solid phases can be used, such as beads formed of glass, polystyrene, polypropylene, dextran or other material. Other suitable solid phases include tubes or microwell plates formed from or coated with these materials.

The recombinant HBLV protein can be either covalently or non-covalently bound to the solid phase by techniques such as covalent bonding via an amide or ester linkage or adsorption. After the protein is affixed to the solid phase, the solid phase can be post-coated with an animal protein, e.g., 3% fish gelatin. This provides a blocking protein which reduces nonspecific adsorption of protein in the sample to be tested to the immunoadsorbent surface.

The immunoadsorbent functions to insolubilize anti-HBLV antibody in the liquid sample tested. In blood screening for anti-HBLV antibody, the immunoadsorbent is incubated with blood plasma or serum. Before incubation, plasma or serum is diluted with normal animal plasma or serum. The diluent plasma or serum is derived from the same animal species that is the source of the anti-(human IgG) antibody. The preferred anti-(human IgG) antibody is goat anti-(human IgG) antibody. Thus, in the preferred format, the diluent would be goat serum or plasma. The optimal dilution factor for human plasma and serum is about 10-11 fold.

The conditions of incubation, e.g. pH and temperature, and the duration of incubation are not crucial. These parameters can be optimized by routine experimentation. Generally, the incubation will be run for 1-2 hours at about 45°C in a buffer of pH 7-8.

After incubation, the immunoadsorbent and the sample are separated. Separation can be accomplished by any conventional separation technique such as sedimentation or centrifugation. The immunoadsorbent then may be washed free of sample to eliminate any interfering substances.

To assess human antibody bound to the immunoadsorbent, the immunoadsorbent is incubated with the labeled anti-(human IgG) antibody (tracer). Generally, the immunoadsorbent is incubated with a solution of the labeled anti-(human IgG) antibody which contains a small amount (about 1%) of the serum or plasma of the animal species which serves as the source of the anti-(human IgG) antibody. Anti-(human IgG) antibody can be obtained from any animal source. However, goat anti-(human IgG) antibody can be an antibody against the F_{c} fragment of human IgG, for example, goat anti-(human IgG) F_{c} antibody.

The anti-(human IgG) antibody or anti-(human IgG)F_{c} can be labeled with a radioactive material such as ¹²⁵Iodine; labeled with an optical label, such as a flourescent material; or labeled with an enzyme such as a peroxidase. The anti-human antibody can also be biotinylated and labeled avidin used to detect its binding to the immunoadsorbent.

After incubation with the labeled antibody, the immunoadsorbent is separated from the solution and the label associated with the immunoadsorbent is evaluated. Depending upon the choice of label, the evaluation can be done in a variety of ways. The label may be detected by a gamma counter if the label is a radioactive gamma emitter, or by a fluorimeter, if the label is a fluorescent material. In the case of any enzyme label detection may be done colorimetrically employing a chromogenic substrate for the enzyme. For example, the enzyme horse radish peroxidase can be used in conjunction with the chromogenic substrate O-Phenylenediamine-2HCl.

The amount of label associated with the immunoadsorbent is compared with positive and negative controls in order to determine the presence of anti-HBLV antibody. The controls are generally run concomitantly with the sample to be tested. A positive control is a serum containing antibody against HBLV protein; a negative control is a serum from uninfected individuals which do not contain antibody against HBLV protein.

For convenience and standardization, reagents for the performance of the assay can be assembled in assay kits. A kit for screening blood for anti-HBLV antibody, for example, can include (in separate containers):
a) an immunoadsorbent e.g. a polystyrene bead coated with a recombinant HBLV protein (preferably the protein encoded by ORF3 as shown in figure 3);
b) an anti-(human IgG) antibody e.g. goat anti-(human IgG) antibody in buffered, aqueous solution containing about 1% goat serum or plasma;
c) a diluent for a serum or plasma sample, e.g. normal goat serum or plasma;
d) a positive control i.e. serum containing antibody against HBLV; and
e) a negative control e.g. pooled sera from healthy individuals which does not contain antibody against HBLV.
If the label is an enzyme, an additional element of the kit can be the substrate for the enzyme.

Another type of assay for anti-HTLV-III antibody is an antigen sandwich assay. In this type of an assay, a labeled HBLV recombinant protein is used in place of anti-(human IgG) antibody to detect anti-HBLV antibody bound to the immunoadsorbent. The assay is based in principle on the bivalency of antibody molecules. One binding site of the antibody binds the antigen affixed to the solid phase; the second is available for binding the labeled antigen. The assay procedure is essentially the same as described for the immunometric assay except that after incubation with the sample, the immunoadsorbent is incubated with a solution of labeled core polypeptide. The HBLV protein can be labeled with radioisotope, an enzyme, etc. for this type of assay.

In a third format, the bacterial protein, Protein A, which binds the F_{c} segment of an IgG molecule without interfering with the antigen-antibody interaction can be used as the labeled tracer to detect antibody adsorbed to the immunoadsorbent. Protein A can be readily labeled with a radioisotope, enzyme or other detectable species.

Immunochemical assays employing recombinant HBLV proteins for detection of antibodies against HBLV protein have several advantages over those employing a whole (or disrupted) virus for this purpose. For one, assays based upon the recombinant proteins will alleviate the concern over growing large quantities of infectious virus and the inherent variability associated with cell culturing and virus production. Efficient expression of viral antigens in E. coli as other host cell systems provide a safe means of large scale production of assay reagents. Further, the assay will help mitigate the real or perceived fear of contracting HBLV infection by technicians in hospitals, clinics and blood banks who perform the test. As mentioned, reagents for assays based upon the whole virus (e.g. whole virus antigen immunoadsorbent), even though they are made with a disrupted, inactivated virus, present a risk of contamination with live virus. For example, a possible source of live virus contamination may be residual cell debris from the virus isolation process. Although extensive precautions can be taken to reduce the risk of contamination, it is virtually impossible to completely eliminate it. Significantly, the risk, though minimal, may be perceived as greater than it actually is by persons who handle the test reagents. Assays reagents without whole virus can help minimize this perception of risk.

The HBLV proteins of this invention may be active in stimulating humoral and/or cellular immune response to HBLV in an individual. For this purpose, immunogen compositions can be prepared comprising an immunizing amount of an immunoactive HBLV protein (preferably a protein encoded by the ORF3 of the 9kb HindIII segment of the HBLV genome, and especially the protein having the sequence set forth in figure 2) and a physiologically acceptable vehicle (e.g., a buffer). In addition, the immunogen composition can contain minor amounts of auxiliary substances such as aluminum hydroxide, which enhance the effectiveness of the composition. The immunogens are administered parenterally, by injection, for example, either subcutaneously or intramuscularly.

Alternatively, the HBLV/vaccinia virus recombinants can be formed which express the HBLV proteins. For example, the ORF3 sequence of figure 3 can be recombined with vaccinia virus to provide a live vaccine against HBLV. See U.S. Patent No. 4,603,112, Paoletti, et al.

The invention is illustrated further by the following Exemplification.

### Exemplification

### Methods

### 1. Construction of the expression vector containing the HBLV DNA fragments.

The 9 kb HindIII HBLV DNA fragment was excised from pZVH14 by restriction endonuclease HindIII and recovered from the low melting point agarose gel. L.H. Guo and R. Wu, Methods in Enzymology 100, 60 (1983). Fragments of this HBLV DNA were generated by two methods: (i) The 9 kb HBLV DNA was digested with restriction endonuclease EcoRI and treated with exonuclease Ba131 in the presence of 20 mM Tris-HC1 (pH 7.5), 600 mM NaC1, 12.5 mM MgC1, and 1 mM EDTA for 2, 4, or 6 min; (ii) The 9 kb fragments were ligated using T4 DNA ligase to form linear or circular fragments of much larger lengths and then sonicated in the presence of 100 mM Tris-HC1 (pH 7.5), 10 mM EDTA. These randomly sheared DNA fragments were electrophoressed on a low melting point agarose gel and fragments from 300 bp to 1000 bp in length were recovered from the gel. See, L.H. Guo and R. Wu, supra. The HBLV fragments prepared by the above two methods were then ligated to NruI-cleaved pMLB1111 with T4 DNA ligase and used to transform E. coli MC1061. Lac⁺ colonies were screened on MacConkey agar plates and picked for further studies.

### 2. Mapping of the open-reading-frames on the HBLV subgenomic DNA

The DNA inserts of the Lac⁺ clones which produced hybrid protein bands on the SDS-PAGE gel were mapped using colony hybridization method as described by M. Grunstein and D. Hogness, Proc. Natl. Acad. Sci. USA 72, 3961 (1975). The probe was prepared as follows: The 9 kb HindIII fragment derived from pZVH14 was digested with restriction endonuclease EcoRI or Bam HI or both and then filled in with [α-³²P] dATP using Klenow fragment. The [α-³²P] labelled DNA fragments were separated by electrophoresis on 1.5% agarose gels. The portions of agarose gel containing the individual ³²P labelled DNA fragment were cut out from the gel and the specific ³²P labelled DNA probes were obtained by adding 200 ul of TE buffer (10 mM Tris-HC1 (pH 7.5), 1 mM EDTA) to the gel pieces and boiled at 100°C for 10 min to denature the DNA. Hybridizations were performed at 67°C in buffers containing 1.5x SSPE (100 mM NaC1, 10 mM sodium phosphate (pH 7.0), 10 mM EDTA), 1% SDS and 0.5% of non-fat dry milk. Clones that hybridized with various probes were compared and divided into four separate noncross-reactive regions designated as ORF 1-4. Three clones from each group were picked randomly and grown up. Plasmid DNA from these clones were isolated and analyzed to determine the insert sizes and locations and to map various restriction enzyme cleavage sites. Several clones containing junctional DNA segment between the ORFs were prepared. The HBLV DNA inserts from all these clones were partially sequenced to determine the sequences of the junctional regions of the HBLV inserts and to drop specific reading frame on HBLV DNA. The arrows labelling pHBLV5 and 6 in figure 2 specify the locations and sizes of the HBLV DNA inserts contained in the two clones. They also define the transcriptional orientation of the encoded genes as from left to right. The wave lines define the approximate boundaries of the 4 ORF's. The open boxes are the T₃ and T₇ contained on the vector Bluescribe of pZVH14. The arrows indicate the transcriptional direction of these two promoters.

### 3. SDS-polyacrylamide gel electrophoretic and Wester immunoblotting analyses of HBLV- -galactosidase fusion proteins

Plasmid pHBLV6 or pMLB1115 were grown in 1 liter L-broth containing 100 µg/ml ampicillin at 37°C for 14 hrs. Bacterial cells were collected by centrifugation and resuspended in 100 mM Tris-HC1 buffer (pH7.4), containing 10 mM EDTA and 1 mM phenylmethylsulfonylfluoride. Cells were disrupted using a French press and the cell debris removed by centrifugation. The protein in the supernatant were precipitated with saturated ammonium sulfate solution. Half of the precipitated proteins were further purified by immunoaffinity chromatography using CNBr-activated Sepharose 4B conjugated with monoclonal anti- β-galactosidase. W. B. Jakoby and M. Wilchek, Method in Enzymology 34 (1975). In (A), protein samples were analyzed on 7.5% SDS-polyacrylamide gel and electrotransferred onto a nitrocellulose paper and stained with amido black. Lane 1 and 3 were unpurified protein extracted from pHBLV6 and pMLB1115, respectively. Lane 2 and 4 were purified protein sample from pHBLV6 and pMLV1115 (lane 6 and 8) were separated on 7.5% SDS-PAGE and transferred onto nitrocellulose filter paper as in (A). The nitrocellulose paper was incubated for 1 hr at room temperature with 5% nonfat dry milk, 0.1% Antifoam A, and 0.1% thimerosal in Tris-buffered saline (pH 7.5). Normal donor serum (lane 5 and 6) and HBLV-infected patient serum (lane 7 and 8) at the dilution of 1:100 were added and incubated at 4°C overnight. The sera had been preabsorbed with E. coli lysate and purified β-galactosidase at 4°C overnight. After washing with Tris-buffered saline (pH 7.5) containing 0.05% Tween 20 for 30 min. at room temperature for three times, the nitrocellulose filter strips were incubated with peroxidase conjugated goat anti-human IgG at room temperature for 1 hr. washed and reacted with the substrate 4-chloro-1-napthol and 0.015% hydrogen peroxide.

### Results

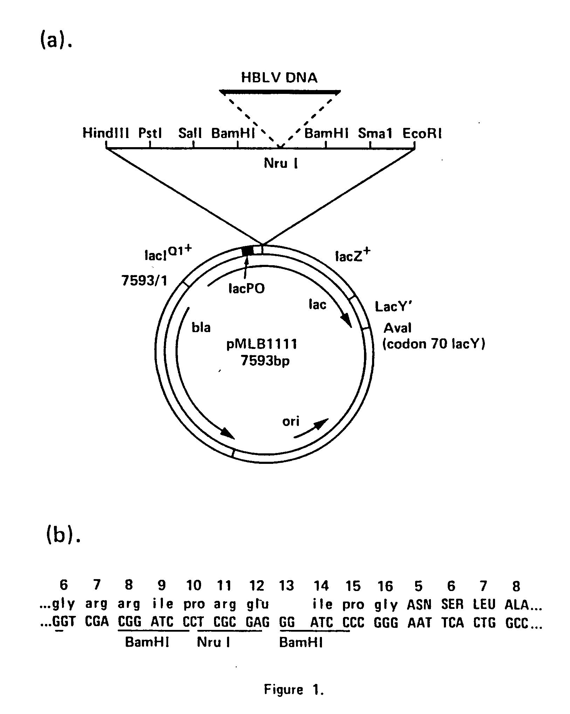

Two different methods were used to generate fragments from the DNA derived from pZVH14. In one method, the 9 kb HindIII HBLV fragment from pZVH14 was first digested with restriction endonuclease EcoRI and then treated briefly with exonuclease Ba131; in the other, the 9 kb HindIII fragment was first self-ligated and then sonicated to generate randomly sheared DNA fragments. The fragments were separated by electrophoresis on an agarose gel and those of 300 to 1000 bp long were isolated. The purified DNA fragments were used to construct plasmids employing an open-reading-frame expression vector pMLB1111 (Figure 1). pMLB1111 contains multiple cloning sites on a polylinker DNA segment inserted near the 5'-end of the wild type LacZ gene. The insertion of this polylinker causes a frame-shift mutation in the Lacz gene. Thus pMLB1111 does not produce any functional -galactosidase when introduced into the E. coli MC1061 [r⁻m⁺ (Δlac) U169] host. M. Casadaban and S. Cohen, J. Mol. Biol., 138, 179 (1980). However, a foreign DNA containing an open-reading-frame can reverse the frame-shift mutation, providing that it is a continuous open-reading-frame of 3N+1 bps (N being an integral) in-phase at its 5' and 3' ends with the divided LacZ gene. The E. coli transformants of the plasmids will produce fusion proteins with the polypeptides encoded by the foreign DNA's inerted between the two β-galactosidase peptides encoded by the split LacZ gene segments. The enzymatic activity of β-galactosidase in the fusion protein is retained.

Transformants were screened on MacConkey agar plates to detect in situ individual clones that express β-galactosidase activity. About 3,100 ampicillin-resistant Lac⁺ transformants were obtained. When these clones were further analyzed by colony hybridization [8] using [α-³²P] dATP-labelled nick-translated 9 kb HindIII fragment from pZVH14 (P.W. Rigby et al., J. Mol. Biol. 113, 237 (1977)) as a probe, approximately 30% of them were found to contain DNA inserts from the 9 kb HindIII fragment. The remaining Lac⁺ transformants probably arose from frame-shift reversion mutations created by imprecise restriction enzyme cleavage by NruI of the pMLB1111 DNA or from the insertion of HBLV DNA fragments which are too small to be detected by the colony hybridization method.

To analyze the HBLV DNA inserts in these Lac⁺ transformants and to determine their locations in pZVH14 DNA, we hybridized these clones via colony hybridization methods employing various ³²P-labelled restriction DNA fragments derived from separate regions of pZVH14 as the probes. These HBLV DNA containing Lac⁺ clones were segregated into four separate non-cross-reactive groups. Additional analyses by DNA sequencing of the inserted HBLV DNA's in these Lac⁺ transformants and of pZVH14 DNA (data not shown) suggested that there probably exist four regions containing open-reading-frame coding sequences on pZVH14, as shown in Figure 2.

The proteins produced by the Lac⁺ transformants were analyzed by sodium dodecyl sulfate polyacrylamide gel electrophoresis (SDS-PAGE) (U.K. Laemli, Nature, London 227:68 (1970)) along with that from the control Lac⁺ bacteria bearing pMLB1115 (obtained from M.L. Berman; see G.M. Weinstock et al. PNAS 80:4432). The linker DNA inserted at the N-terminus of the LacZ gene in pMLB1115 is identical to that of pMLB1111 except that it contains an extra base which renders the insertion linker in-phase with the LacZ gene. Thus, bacteria harboring pMLB1115 produce functional β-galactosidase. By virtue of their very large size, β-galactosidase and its fusion proteins are separated on SDS-polyacrylamide gels from the bulk of proteins in cell lysates and can be easily identified by Coomassie brilliant blue staining (Figure 3A).

One hundred and twenty Lac⁺ clones were analyzed by SDS-PAGE and 67 of them produced polypeptides larger by 15,000 to 35,000 daltons than the intact β-galactosidase produced in pMLB1115. The findings about their sizes are consistent with data indicating that the sizes of the inserted HBLV DNA's in these Lac⁺ transformants are 300-1000 bps in length. The rest of the Lac⁺ clones analyzed produced a protein with electrophoretic mobility similar to that of native β-galactosidase. This may be due to the very small size of some HBLV DNA inserts or to certain proteolytic degradation of the fusion protein, as observed previously with a similar expression system. See N.T. Chang et al., Science 228, 93, (1985). Judged by the color intensities of the Coomassie Blue-stained protein bands, the expressed HBLV- β-galactosidase fusion protein accounts for about 0.5 to 1 percent of the total cellular protein.

The immunoreactivities of the expressed HBLV-β-galactosidase fusion proteins were examined by Western blot techniques using sera from patients. These sera were selected because they had previously been found to contain antibodies against HBLV, as determined by their specific reactivity with HBLV-infected lymphocytes in culture using immunofluorescence staining techniques. S.Z. Salhuddin et al. supra. In addition, HBLV had been isolated or identified in those patients from whom the sera were obtained.

Of the 67 Lac⁺ clones which contain fusion proteins larger by 15,000 to 35,000 daltons than the intact β-galactosidase analyzed by Western blot, the fusion proteins from 2 clones (pHBLV5 and pHBLV6) reacted specifically with sera from the HBLV infected patients (Figure 3B). One serum was strongly immunoreactive with the recombinant protein, and the other two weakly reactive. Early studies of these patients' antibodies on their immunofluorescent staining of HBLV-infected cultured lymphocytes gave parallel results. S.Z. Salahuddin et al., supra. It is possible that the different reactivities of these sera with the recombinant HBLV proteins are due to different affinities or to concentrations of the HBLV-specific antibodies present in the sera.

DNA hybridization data showed that the two clones were derived from a single open-reading-frame region designated as ORF3. These results suggest that the gene containing ORF3 is expressed in patients infected with HBLV and the proposed protein is immunogenic. Clone pHBLV6 was chosen for further analyses, because it produced a higher level of the recombinant HBLV peptide. The recombinant fusion protein in cell lysates was affinity-purified using a column of Sepharose-4B conjugated with a mouse monoclonal antibody specific for β-galactosidase. See W.B. Jakoby and M. Wilchek, Methods in Enzymology 34 (1975).

The protein purified from pHBLV6 contains four distinctive Coomassie brilliant blue stained bands on SDS-polyacrylamide gels. The peptide in one of the two major bands has a molecular weight of approximately 150 kilodaltons (kd), which corresponds to that of the HBLV-β-galactosidase fusion protein. The other major polypeptide is about 120 kd, similar in size to the native β-galactosidase. Since the 150 kd band is immunoreactive with sera from HBLV-infected patients and the 120 kd is not, the 120 kd band is probably derived from the 150 kd HBLV- β-galactosidase fusion protein by partial degradation in the bacterial host cells and hence contain little HBLV-encoded moiety. The 135 kd minor species is probably another partially degraded HBLV- β-galactosidase intermediate polypeptide. The nature of the 155 kd species is unknown at the present time. Since the 155 kd and 135 kd proteins are not reactive with the patients' sera (Figure 3, lane 2 and 7), it is possible that both these proteins may be contaminants of E. coli origin that are co-purified with the recombinant protein by the affinity column. Further purification of the HBLV proteins is in progress to clarify the biochemical nature of the various proteins.

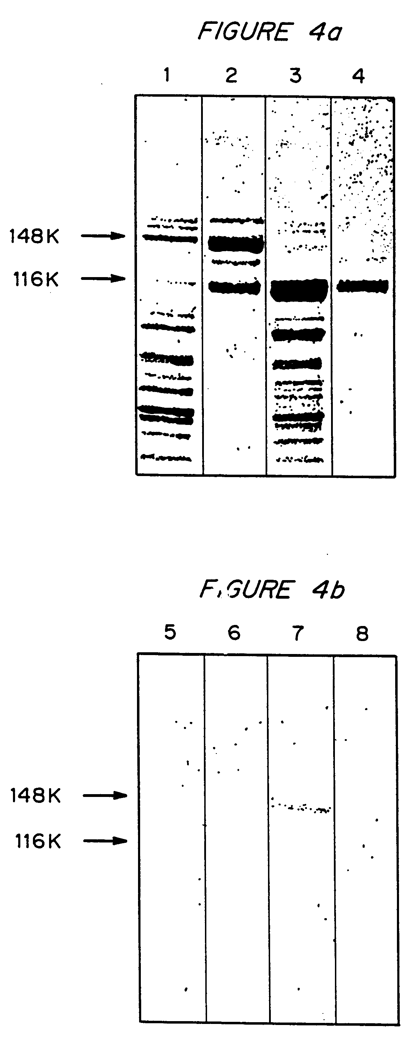

The HBLV DNA inserted in pHBLV6 was sequenced by dideoxy chain termination methods. E.Y. Chen and P.H. Seeburg, DNA 4, 165 (1985). The insert of pHBLV6. (Figure 4). The results indicate that there exists a single continuous coding frame on one of three DNA reading frames present on pHBLV6. Thus, the DNA segment encompassed by pHBLV6 is probably a portion of a structural gene. Comparison of this DNA sequence with all known DNA sequences in a DNA data bank, Gene Bank, revealed that the HBLV DNA is novel and has no significant sequence homology with any known DNA so far sequenced. Hydropathicity analysis (J. Kyle and R.F. Doolittle, J. Mol. Biol., 157, 105 (1982)) of the deduced polypeptide sequence indicate that the pHBLV6-encoded HBLV peptide is hydrophilic. In addition, the peptide contains no potential N-link glycosylation sites and no stretches of hydrophobic residues. Glycosylation of a viral peptide usually would indicate that the peptide is a coat or envelope protein and the presence of a stretch of hybrophobic amino acid residues (often in the neighborhood of 15 residues) and would suggest that the peptide spans through cellular plasma membrane. The secondary structure as prediced is mainly helical structure. It is not yet possible to tell the location of the viral protein specified partly by pHBLV-6 in the virions and in the infected cells.

We have also performed additional DNA sequencing analyses on a number of clones derived from the ORF2, ORF3 and ORF4 regions and on the junctional regions of pZVH14 DNA spanning between the assigned ORFs (data not shown). Although the results are not yet sufficient to define the exact boundaries of the individual open-reading-frames, they confirm our earlier suggestion that ORF2 and ORF3 are derived from different reading frames on the HBLV DNA and probably encode separate genes of the virus. The overlapping ORF3 and ORF4 are in-frame with each other and possibly derived from a very long (over 3 kb) open-reading-frame (ORF3+4) capable of encoding a polypeptide larger than 100 kd.

### Deposit

The clonal cell line designated pHBLV6 was deposited at the American Type Culture Collection (ATCC), Rockville, Maryland 20852 USA on June 1, 1987. The ATCC designation is 67423. The deposit was made under the provisions of the Budapest Treaty.

## Claims

1. An isolated HBLV protein which is immunoreactive with antibody against HBLV comprising an amino acid sequence encoded by (1) the ORF3 of Fig. 2, or (2) immunological equivalents thereof which are created by deletion, insertion or substitution of the ORF3 of figure 2.

2. The protein of claim 1 comprising the amino acid sequence of Figure 3

3. DNA encoding the protein of claim 1 or claim 2, or coding equivalents thereof.

4. A recombinant expression vector eg. a plasmid containing in expressible form the DNA of Claim 3.

5. The expression vector of claim 4 which encodes a fusion protein e.g. a β-galactosidase fusion protein.

6. The expression vector of claim 5 which is pHBLV6, as obtainable from cell line ATCC 67423.

7. A cell transformed with the expression vector according to any one of Claims 4-6.

8. A bacterial cell eg. an E. coli cell transformed with a recombinant plasmid, the plasmid comprising prokaryotic transcriptional and translational signals linked to DNA according to Claim 3.

9. A method of producing HBLV immunoreactive protein comprising the steps of:
a. transforming a host cell eg. a bacterial cell with a recombinant vector eg. a plasmid containing in expressible form DNA according to Claim 3;
b. culturing the transformed host cell; and
c. isolating the protein from the host cell.

10. A method of detecting antibody against HBLV in a biological fluid eg. human serum or plasma, comprising the steps of:
a. providing an antigen immunoadsorbent comprising a solid phase to which is attached an HBLV protein according to Claim 1 or Claim 2;
b. incubating the immunoadsorbent with a sample of the biological fluid to be tested under conditions which allow antibody in the sample to complex with the antigen immunoadsorbent;
c. separating the immunoadsorbent from the sample; and
d. determining antibody bound to the immunoadsorbent as an indication of antibody against HBLV in the sample.

11. A method of Claim 10, wherein the step of determining the antibody bound to the immunoadsorbent comprises:
a) incubating the immunoadsorbent with a labeled antibody against immunoglobulin of the species from which the biological fluid is derived eg. labeled anti-human Ig antibody;
b) separating the immunoadsorbent from the labeled antibody; and
c) detecting the label associated with the immunoadsorbent as an indication of antibody against HBLV in the sample.

12. An immunoadsorbent comprising a solid phase support having attached thereto an HBLV protein according to Claim 1 or Claim 2.

13. A kit for performance of an immunoassay for detection of HBLV antibodies, comprising:
a. an immunoadsorbent according to Claim 12; and
b. labeled anti-human Ig.

14. A kit according to Claim 13, further comprising
c. a diluent for a sample to be tested;
d. a positive control; and
e. a negative control.

15. An immunogen composition comprising an immunogenic amount of HBLV protein according to Claim 1 or Claim 2 and a physiologically acceptable vehicle.

## Patentansprüche

1. Isoliertes HBLV-Protein, das in bezug auf Antikörper gegen HBLV immunoreaktiv ist, das (1) eine durch ORF3 gemäß Figur 2 codierte Aminosäuresequenz aufweist oder (2) immunologische Äquivalente hiervon, die durch Deletion, Insertion oder Substitution von ORF3 gemäß Figur 2 erhältlich sind.

2. Protein gemäß Anspruch 1, das die Aminosäuresequenz gemäß Figur 3 enthält.

3. DNA, die das Protein gemäß Anspruch 1 oder Anspruch 2 codiert, oder codierende Äquivalente hiervon.

4. Rekombinanter Expressionsvektor, z.B. ein Plasmid, der die DNA gemäß Anspruch 3 in exprimierbarer Form enthält.

5. Expressionsvektor gemäß Anspruch 4, der für ein Fusionsprotein codiert, z.B. ein β-Galactosidasefusionsprotein.

6. Expressionsvektor gemäß Anspruch 5, der pHBLV6 darstellt, wie er aus der Zellinie ATCC 67423 erhältich ist.

7. Zelle, die mit dem Expressionsvektor gemäß einem der Ansprüche 4 bis 6 transformiert wurde.

8. Bakterienzelle, z.B. eine E. coli-Zelle, die mit einem rekombinanten Plasmid transformiert wurde, wobei das Plasmid prokariotische transkriptionelle und translationelle Signale aufweist, die mit der DNA gemäß Anspruch 3 verbunden sind.

9. Verfahren zur Herstellung eines HBLV immunoreaktiven Proteins, das folgende Schritte aufweist:
a) Transformieren einer Wirtszelle, z.B. einer Bakterienzelle, mit einem rekombinanten Vektor, z.B. einem Plasmid, das die DNA gemäß Anspruch 3 in exprimierbarer Form enthält;
b) Kultivieren der transformierten Wirtszelle;
c) Isolieren des Proteins aus der Wirtszelle.

10. Verfahren zum Nachweis eines Antikörpers gegen HBLV in einer biologischen Flüssigkeit, z.B. menschlischem Serum oder Plasma, das folgende Schritte aufweist:
a) Bereitstellen eines antigenen Immunoadsorbens, das eine feste Phase aufweist, an die ein HBLV-Protein gemäß Anspruch 1 oder Anspruch 2 angeheftet ist;
b) Inkubieren des Immunoadsorbens mit einer Probe der zu untersuchenden biologischen Flüssigkeit unter Bedingungen, die es erlauben, Antikörper in der Probe mit dem antigenen Immunoadsorbens zu komplexieren;
c) Trennen des Immunoadsorbens von der Probe; und
d) Bestimmen des Antikörpers, der an das Immunoadsorbens gebunden ist, als Hinweis auf Antikörper gegen HBLV in der Probe.

11. Verfahren nach Anspruch 10, wobei der Schritt des Bestimmens des an das Immunoadsorbens gebundenen Antikörpers beinhaltet:
a) Inkubieren des Immunoadsorbens mit einem markierten Antikörper gegen Immunoglobulin derjenigen Spezies, von der die biologische Flüssigkeit stammt, z.B. markiertem anti-human Ig Antikörper;
b) Trennen des Immunoadsorbens von dem markierten Antikörper;
c) Nachweisen der Markierung, die mit dem Immunoadsorbens assoziiert ist, als Hinweis von Antikörpern gegen HBLV in der Probe.

12. Immunoadsorbens, das als feste Phase einen Träger aufweist, der hieran gebunden ein HBLV-Protein gemäß Anspruch 1 oder 2 besitzt.

13. Kit zur Durchführung eines Immunoessays zum Nachweis von HBLV-Antikörpern, enthaltend:
a) ein Immunoadsorbens gemäß Anspruch 12; und
b) markiertes anti-human Ig.

14. Kit gemäß Anspruch 13, weiterhin enthaltend:
c) ein Verdünnungsmittel für die zu untersuchende Probe;
d) eine positive Kontrolle; und
e) eine negative Kontrolle.

15. Immunogene Zusammensetzung, die eine immunogene Menge von HBLV-Protein gemäß Anspruch 1 oder Anspruch 2 und ein physiologisch verträgliches Medium enthält.

## Revendications

1. Une protéine de HBLV isolée, qui est immunoréactive en présence d'un anticorps dirigé contre HBLV, comprenant une séquence d'acides aminés codée par (1) l'ORF3 de la figure 2, ou (2) des équivalents immunologiques de celui-ci, qui sont créée par suppression, insertion ou substitution de l'ORF3 de la figure 2.

2. La protéine de la revendication 1, comprenant la séquence d'acides aminés de la figure 3.

3. L'ADN codant pour la protéine de la revendication 1 ou de la revendication 2, ou codant pour des équivalents de celle-ci.

4. Un vecteur d'expression recombinant, par exemple un plasmide contenant, sous une forme exprimable, l'ADN de la revendication 3.

5. Le vecteur d'expression de la revendication 4, qui code pour une protéine de fusion, par exemple pour une protéine de fusion β-galactosidase.

6. Le vecteur d'expression de la revendication 5, qui est pHBLV6, tel que l'on peut l'obtenir à partir de la lignée cellulaire ATCC 67423.

7. Une cellule transformée par le vecteur d'expression, conformément à l'une quelconque des revendications 4-6.

8. Une cellule bactérienne, par exemple une cellule d' E. coli transformée par un plasmide recombinant, le plasmide comprenant des signaux procaryotes transcriptionnels et translationnels liés à l'ADN selon la revendication 3.

9. Une méthode de production d'une protéine immunoréactive de HBLV, comprenant les étapes de:
a. transformer une cellule hôte, par exemple une cellule bactérienne avec un vecteur recombinant, par exemple un plasmide contenant, sous une forme exprimable, l'ADN selon la revendication 3;
b. cultiver la cellule hôte transformée; et
c. isoler la protéine, à partir de la cellule hôte.

10. Une méthode de détection d' un anticorps dirigé contre HBLV, dans un fluide biologique, par exemple du sérum ou du plasma humain, comprenant les étapes de:
a. fournir un immunoadsorbant antigénique comprenant une phase solide à laquelle est attachée une protéine de HBLV selon la revendication 1 ou la revendication 2;
b. incuber l'immunoadsorbant avec un échantillon du fluide biologique à tester, dans des conditions qui permettent à l'anticorps, dans l'échantillon, de se complexer à l'immunoadsorbant antigénique;
c. séparer l'immunoadsorbant de l'échantillon; et
d. déterminer la liaison de l'anticorps à l'immunoadsorbant comme une indication de la présence de l'anticorps dirigé contre HBLV dans l'échantillon.

11. Une méthode de la revendication 10, dans laquelle l'étape de détermination de la liaison de l'anticorps à l'immunoadsorbant comprend:
a) l'incubation de l'immunoadsorbant avec un anticorps marqué dirigé contre une immunoglobuline de l'espèce à partir de laquelle le fluide biologique est dérivé, par exemple un anticorps Ig anti-humain marqué;
b) séparer l'immunoadsorbant de l'anticorps marqué; et
c) détecter le marquage associé à l'immunoadsorbant comme une indication de la présence de l'anticorps dirigé contre HBLV dans l'échantillon.

12. Un immunoadsorbant comprenant un support solide auquel est attachée une protéine de HBLV selon la revendication 1 ou la revendication 2.

13. Un nécessaire, pour l'accomplissement d'un immunoessai pour la détection des anticorps de HBLV, comprenant :
a. un immunoadsorbant conformément à la revendication 12; et
b. un Ig anti-humain marqué.

14. Un nécessaire, selon la revendication 13, comprenant en outre:
c. un diluant, pour un échantillon à tester;
d. un contrôle positif; et
e. un contrôle négatif.

15. Une composition immunogène comprenant une quantité immunogénique d'une protéine de HBLV selon la revendication 1 ou la revendication 2 et un véhicule physiologiquement acceptable.
